Europäisches Patentamt

European Patent Office

Office européen des brevets

(11) Publication number: **0 197 786**
A1

## EUROPEAN PATENT APPLICATION

(21) Application number: 86302549.0

(22) Date of filing: 07.04.86

(51) Int. Cl.⁴: **G 01 T 1/161**
G 01 T 1/00, A 61 B 5/02

(30) Priority: 10.04.85 GB 8509150
24.09.85 GB 8523514

(43) Date of publication of application:
15.10.86 Bulletin 86/42

(84) Designated Contracting States:
AT BE CH DE FR GB IT LI NL SE

(71) Applicant: Lahiri, Avijit
22 Aston Avenue
Kenton Harrow Middlesex(GB)

(72) Inventor: Crawley, John Cornelius William
189 Handside Lane
Welwyn Garden City Hertfordshire(GB)

(72) Inventor: Caunt, John Francis
37 Newland Street
Eynsham Oxfordshire(GB)

(74) Representative: Rushton, Ronald et al,
SOMMERVILLE & RUSHTON 11 Holywell Hill
St. Albans Hertfordshire AL1 1EZ(GB)

(54) Improvements relating to cardiac and the like monitoring.

(57) A probe for use in the characterisation of bloodflow in the heart or other tissue by measurement of radioactivity from a tracer injected into the bloodstream comprises a crystal (1) adapted to scintillate in response to gamma radiation incident from the tissue under investigation, and a solid state photodiode (2) optically coupled to the crystal to derive an electrical signal indicative of the incident radiation. The photodiode replaces the large and cumbersome photomultiplier tube of conventional nuclear bloodflow detectors and results in a useful miniaturisation to facilitate e.g. ambulatory monitoring, and an overall improvement in convenience of operation.

Fig. 1.

EP 0 197 786 A1

Croydon Printing Company Ltd.

-1-

## Improvements relating to cardiac and the like monitoring

The present invention relates to an apparatus and method for use in the non-invasive characterisation of bloodflow in body tissue by measurement of radioactivity from a tracer injected into the patient's bloodstream.   In particular, the invention is concerned with the measurement of left ventricular ejection fraction and other related heart performance parameters, although apparatus in accordance with the invention may also find utility in monitoring the uptake of isotope tracers by organs or other tissue other than the heart.

Techniques of this kind have been practised for some years, one known apparatus which is capable of beat-to-beat monitoring of cardiac bloodflow parameters by the radioactive blood-pool technique being known as the Nuclear Stethoscope, manufactured by Bios Inc of Valhalla, NY, USA.   This apparatus includes a lead-collimated detector or probe comprising a thallium-activated sodium iodide crystal typically 5 cm in diameter and 3.5cm thick followed by a 5cm diameter photomultiplier tube typically 25cm long.   In use, the probe is located adjacent to the patient's chest above the left ventricle and gamma radiation received by the crystal from the labelled blood in the region of tissue under investigation - technetium - 99m is the usual tracer - causes the crystal to scintillate, the resultant light photons being converted into a stream of electrons and amplified by the photomultiplier tube.

The output from the probe is fed into an amplifier for pulse shaping and amplification and thence into a pulseheight analyser (threshold discriminator)which discriminates between the desired isotope photopeak and scattered radiation and electronic noise. The logic pulses from the discriminator are input to a dedicated microcomputer which is programmed to calculate and display the required cardiac parameters in conjunction with synchronisation signals derived from the patient's ECG. Calibration of the apparatus prior to measurement is achieved by first directing the probe to a portion of the patient's chest away from the heart, to detect his level of "background" radiation.

While this known apparatus is capable of providing excellent results under favourable working conditions it does have certain drawbacks. In particular the probe, owing to the size of the crystal and, especially, of the photomultiplier, is relatively large, heavy and cumbersome and intrudes undesirably into the space around the patient. It is also consequently quite unsuitable for direct attachment to the patient's chest wall which means that constant re-positioning of the probe is required for continuous monitoring or exercise studies and rules out any possibility of ambulatory monitoring. The operation of this probe also demands the provision of a high voltage electrical supply. It is one aim of the present invention, therefore, to provide a probe which can be used as the radiation detector in labelled blood-pool monitoring techniques which is amenable to construction in a miniaturised form as compared with the conventional Nuclear Stethscope probe described above.

Accordingly, in one aspect the present invention resides in a probe for use in the characterisation of bloodflow in body tissue by measurement of radioactivity from an injected tracer, comprising a radiation-sensitive crystal adapted to scintillate in response to gamma radiation incident from the tissue under investigation and a solid state photodiode optically coupled to the crystal whereby to derive an electrical signal indicative of said incident radiation. Such a probe may in use be coupled via a suitable charge-sensitive pre-amplifier, pulse-shaping amplifier and threshold discriminator to a microcomputer for calculation of the selected bloodflow parameters in response to the nuclear activity detected by the probe.

By thus eliminating the photomultiplier tube, which contributes the major portion of the size and weight (and expense) of the conventional probe described above, the present invention can achieve an advantageous and very significant miniaturisation in probe design without, however, significantly compromising the accuracy of the bloodflow parameters computed from its output. Operation at low voltage is also made possible.

The invention will now be more particularly described, by way of example, with reference to the accompanying drawings, in which:

Figure 1 is a schematic cross-sectional view of a nuclear probe made in accordance with the invention, showing the general layout of its internal components; and

Figure 2 is a block diagram of the probe and associated apparatus for monitoring bloodflow parameters.

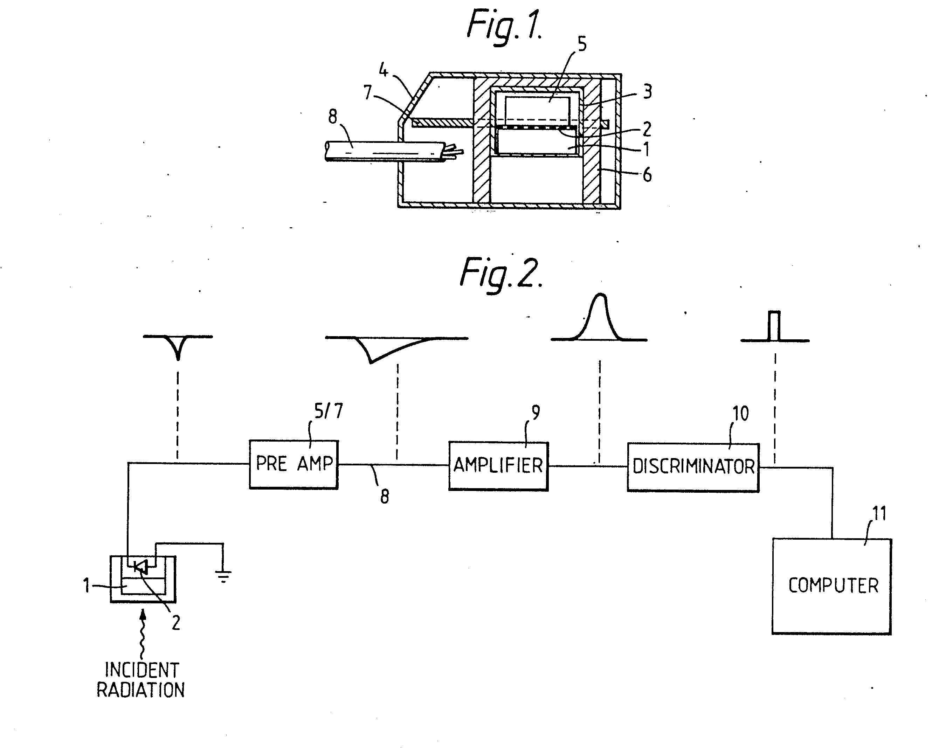

The probe shown in Figure 1 comprises a small - typically 1cm across - scintillation crystal 1 bonded and optically coupled to a solid state photodiode 2, and hermetically sealed within an aluminium can 3 to exclude all ambient light. The crystal 1 may be of thallium-activated sodium iodide or thallium-activated caesium iodide, both of which have been found to work well in probes made in accordance with the invention. The probe is constructed with an RF-screened plastics housing 4 which contains both the nuclear detector itself and the components of a charge-sensitive pre-amplifier. The most sensitive components of the latter, consisting of a primary FET and the preamplifier feedback components, are connected directly onto the pins of the photodiode 2 and are encapsulated in a unit 5 within the can 3 together with the crystal and diode. The can 3 is itself set in a lead holder 6 which forms both an additional electrical screen and a collimator to ensure that incident radiation falls only on the front face of the crystal 1. The rest of the pre-amplifier components are on a "doughnut" printed circuit board 7 mounted around the holder 6, and a cable 8 brings power to the pre-amplifier and takes the signal output from the probe.

In use, the housing 4 is mounted so as to position the crystal 1 above the area of tissue to be investigated and the probe is connected as shown in Figure 2. Scintillation of the crystal 1 in response to gamma radiation from labelled blood in the tissue under investigation is seen by the diode 2 which outputs a corresponding train of electrical current pulses. These pulses are lengthened by the probe's own pre-amplifier and fed to an external high gain amplifier 9 which converts them to shaped voltage pulses. The pulses output from this amplifier are compared with threshold values in a pulse height analyser (threshold discriminator) 10 set to produce a logic pulse for each input pulse representative of the blood tracer photopeak, and the logic pulses are fed to a computer 11 - for example into the TTL pulse input circuit of a conventional Nuclear Stethoscope console - for calculation and display of the desired bloodflow parameters. Figure 2 also indicated typical pulse waveforms (unscaled) at the outputs from the photodiode 2, pre-amplifier 5/7 amplifier 9 and discriminator 10 respectively.

Using probes and associated apparatus as described above, left ventricular ejection fractions (EF%) have been computed for a series of patients undergoing gamma camera blood-pool imaging studies. Both sodium iodide and calsuim iodide crystals have been employed in the probes used for these measurements. In each case values of EF% were obtained which differed from the gamma camera results by a mean of less than 1%, which indicates the accuracy with which the miniature detectors according to the invention can be used to measure this parameter. Spectra obtained with a multichannel analyser showed a significant separation between electronic noise and the 140keV photopeak of Tc-99m.

The housing 4 of a probe as illustrated in figure 1 is typically no more than 5cm across and the entire probe weighs no more than 100g. Such probes can be successfully attached to a patient's chest by a simple elastic strap, which has been found sufficient to keep the probe in place during, for example, maximal upright bicycle exercise, and confirms the suitability of the device for ambulatory monitoring.

In the context especially of long term and ambulatory monitoring it may be preferred to connect the output of a probe as described herein not directly to the data-processing hardware but rather via a suitable analogue or digital interface to a miniature data-recorder (such as the Medilog 4-24 or MR10 tape recorder manufactured by Oxford Medical Systems Ltd, Abingdon, England, or an eventual solid-state recorder), for subsequent processing. With such a combination of equipment it will be possible to record beat-to-beat variations in cardiac function continuously over a long period (typically 8 to 24 hours) with the minimum of restriction on the patient's movement.

-6-

CLAIMS

1. A probe for use in the characterisation of bloodflow in body tissue by measurement of radioactivity from an injected tracer, comprising a radiation-sensitive crystal (1) adapted to scintillate in response to gamma radiation incident from the tissue under investigation, and characterised in that a solid state photodiode (2) is optically coupled to the crystal (1) whereby to derive an electrical signal indicative of said incident radiation.

2. A probe according to claim 1 wherein said crystal (1) is of sodium iodide.

3. A probe according to claim 1 wherein said crystal (1) is of caesium iodide.

4. A probe according to any preceding claim wherein a pre-amplifier (5/7) for the electrical output of said photodiode (2) is incorporated in a structural unit together with said crystal (1) and photodiode (2).

5. A probe according to claim 4 comprising a main housing (4); a sealed enclosure (3) mounted within said housing (4) and containing said crystal (1) and photodiode (2); a radiation-resistant structure (6) juxtaposed to said enclosure (3) to define a collimator for incident radiation from the tissue under investigation; and a board (7) for components of said pre-amplifier located within said housing (4) externally of said enclosure (3) and structure (6)

6. A probe according to claim 5 comprising interference-sensitive components of said pre-amplifier (5) mounted within said enclosure (3) and wherein said radiation-resistant structure (6) extends around said enclosure (3) to screen the same.

# Fig.1.

# Fig.2.

PRE AMP 5/7

AMPLIFIER 9

DISCRIMINATOR 10

COMPUTER 11

INCIDENT RADIATION

**European Patent Office**

## DOCUMENTS CONSIDERED TO BE RELEVANT

| Category | Citation of document with indication where appropriate, of relevant passages | Relevant to claim | CLASSIFICATION OF THE APPLICATION (Int Cl 4) |
|---|---|---|---|
| Y | INTERNATIONAL JOURNAL OF APPLIED RADIATION AND ISOTOPES, vol. 32, no. 10, October 1981, pages 719-727, Pergamon Press Ltd., Oxford, GB; J. BOJSEN et al.: "Portable cadmium telluride detectors and their applicability for external measurement of 51Cr-EDTA clearance" * Page 719, column 2, lines 5-11,25-31; page 720, column 1, lines 6-13; figure 1 * | 1,4 | G 01 T 1/161<br>A 61 B 5/02 |
| Y | PATENTS ABSTRACTS OF JAPAN, vol. 9, no. 68 (P-344) [1791], 28th March 1985; & JP - A - 59 200 983 (TOSHIBA K.K.) 14-11-1984 | 1,4 | |
| | | | **TECHNICAL FIELDS SEARCHED (Int Cl 4)** |
| A | Idem | 5,6 | |
| A | NUCLEAR INSTRUMENTS AND METHODS, vol. 67, no. 1, January 1969, pages 93-102, North-Holland Publishing Co., Amsterdam, NL; J.E. BATEMAN: "Some new scintillator-photodiode detectors for high enegery charged particles" * Table 1 * | 1-3 | G 01 T<br>A 61 B |
| | --- -/- | | |

The present search report has been drawn up for all claims

| Place of search | Date of completion of the search | Examiner |
|---|---|---|
| THE HAGUE | 10-06-1986 | DATTA S. |

| | **DOCUMENTS CONSIDERED TO BE RELEVANT** | | | Page 2 |
|---|---|---|---|---|
| Category | Citation of document with indication, where appropriate, of relevant passages | | Relevant to claim | CLASSIFICATION OF THE APPLICATION (Int Cl 4) |
| A | R.C.A. TECHNICAL NOTES, no. 1281, October 1981, Princeton, New Jersey, US; R.D. FAULKNER et al.: "Blood flow monitor for heart monitoring" * Whole article * | | 1,2 | |
| | ----- | | | |
| | | | | TECHNICAL FIELDS SEARCHED (Int Cl 4) |

| The present search report has been drawn up for all claims | | |
|---|---|---|
| Place of search **THE HAGUE** | Date of completion of the search **10-06-1986** | Examiner **DATTA S.** |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82